# EUROPEAN PATENT SPECIFICATION

(11) **EP 2 253 264 B1**
(45) Date of publication and mention of the grant of the patent: **15.05.2013**
(21) Application number: 09719775.0
(22) Date of filing: 10.03.2009
(51) Int. Cl.: A61B 1/12, A61B 1/00

(54) **CLEANING/DISINFECTING TUBE AND ENDOSCOPE CLEANING/DISINFECTING APPARATUS**
REINIGUNGS- UND DESINFEKTIONSSCHLAUCH UND VORRICHTUNG ZUR REINIGUNG UND DESINFEKTION EINES ENDOSKOPS
TUBE DE NETTOYAGE ET DE DESINFECTION, ET APPAREIL DE NETTOYAGE ET DE DESINFECTION D'ENDOSCOPE

(30) Priority: 13.03.2008 JP 2008064650
(43) Date of publication of application: 24.11.2010
(73) Proprietor: Olympus Medical Systems Corp., Tokyo 151-0072 (JP)
(72) Inventor: KAWASE, Takahiko, Tokyo 151-0072 (JP); TEZUKA, Mitsuyoshi, Tokyo 151-0072 (JP)
(74) Representative: Schicker, Silvia
(86) International application number: PCT/JP2009/054568
(87) International publication number: WO 2009/113550

(56) References cited:
- EP-A2- 1 728 466
- WO-A1-00/56203
- FR-A1- 2 803 755
- JP-A- 8 024 813
- JP-A- 10 314 282
- JP-A- 56 045 664
- JP-A- 58 155 836
- JP-A- 58 192 525
- JP-A- 2004 135 946
- US-A- 4 380 530

## Description

### Technical Field

The present invention relates to a cleaning/disinfecting tube for connecting a liquid delivery connector of an endoscope cleaning/disinfecting apparatus to an opening portion provided in an object to be cleaned, and to an endoscope cleaning/disinfecting apparatus including the cleaning/disinfecting tube.

### Background Art

An endoscope is widely used in medical and industrial fields. When an endoscope and attachments thereto (an endoscope and attachments thereto are hereinafter referred to as objects to be cleaned) are cleaned and disinfected, an endoscope cleaning/disinfecting apparatus is used. In the endoscope cleaning/disinfecting apparatus, objects to be cleaned are immersed in a chemical solution, rinsing water, or another suitable liquid.

An endoscope includes therein various channels, such as those for delivering air and water and those into which treatment instruments are inserted. To clean and disinfect an endoscope, it is necessary to fill the channels with a liquid used in the cleaning and disinfecting processes by supplying the liquid into the channels through the opening portions thereof. To clean and disinfect attachments to an endoscope, it is also necessary to immerse the entire surfaces of the attachments in a liquid used in the cleaning and disinfecting processes.

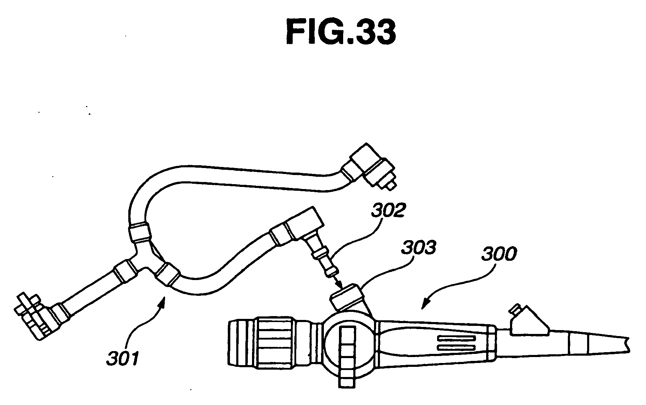

An example of the endoscope cleaning/disinfecting apparatus described above is disclosed in Japanese Patent Application Laid-Open Publication No. 09-253029. In the cleaning/disinfecting apparatus of the related art, a cleaning/disinfecting tube 301 is connected to an opening portion (mouthpiece) of a channel disposed in an endoscope 300, and a liquid is supplied into the channel through the cleaning/disinfecting tube 301, as shown in Fig. 33. A connector section 302 of the cleaning/disinfecting tube 301 is inserted all the way into the mouthpiece and fixed there so that the cleaning/disinfecting tube 301 does not fall off the mouthpiece due to the pressure created when the liquid is supplied.

When a tube connector section of a cleaning/disinfecting tube is inserted into and fixed to an opening portion of an endoscope or an attachment thereto, which is an object to be cleaned, as in the related art, the tube connector section comes into contact with part of the opening portion at all times. Since the portion where the tube connector section comes into contact with the opening portion is unlikely immersed in a liquid used in the cleaning and disinfecting processes, it takes a long time to carry out the cleaning and disinfecting processes, resulting in decrease in efficiency.

The present invention has been made in view of the circumstances described above. An object of the present invention is to provide a cleaning/disinfecting tube that is connected to an opening portion of an object to be cleaned and used to supply a liquid through the opening portion in an endoscope cleaning/disinfecting apparatus, the cleaning/disinfecting tube allowing the entire opening portion to be immersed in the liquid without falling off the opening portion.

Document EP 1 728 466 A discloses a cleaning/disinfecting tube that allows an opening portion provided in an object to be cleaned accommodated in a cleaning tank of an endoscope cleaning/disinfecting apparatus to communicate with a liquid delivery connector which is provided in the endoscope cleaning/disinfecting apparatus and through which a liquid is supplied. The cleaning/disinfecting tube comprises a tube connector section to be connected to the opening portion, and a holding portion that engages with the object to be cleaned with a gap therebetween The holding portion constrains a movement of the tube connector section in a direction away from the opening portion when the tube connector section is connected to the opening portion. In this context, also documents FR 2803755 A, US 4,380,530 A, and WO 00/56203 A should be mentioned which disclose similar cleaning/disinfecting tubes.

### Disclosure of Invention

Means for Solving the Problem

A cleaning/disinfecting tube of the present invention is provided according to claim 1.

### Brief Description of the Drawings

Fig. 1 is an exterior view showing the exterior of an endoscope cleaning/disinfecting apparatus;
Fig. 2 is an exterior view showing the exterior of a sliding tube;
Fig. 3 is a first view showing the configuration of a cleaning/disinfecting tube not part of the present invention;
Fig. 4 is a second view showing the cleaning/disinfecting tube not part of the present invention;
Fig. 5 is a cross-sectional view showing hollow paths in the cleaning/disinfecting tube not part of the present invention;
Fig. 6 is a view for describing the connection of the cleaning/disinfecting tube to the sliding tube;
Fig. 7 is a view showing a state in which an air accumulation is created in the sliding tube;
Fig. 8 is a view showing a state in which a connecting tube is deformed;
Fig. 9 is a view showing a variation of a tube connector section not part of the present invention;
Fig. 10 is a view showing another variation of the tube connector section not part of the present invention;
Fig. 11 is a view showing another variation of the tube connector section not part of the present invention;
Fig. 12 is a view showing a variation of a connecting portion in the first not part of the present invention;
Fig. 13 is a view showing another variation of the connecting portion not part of the present invention;
Fig. 14 is a view showing the configuration of a holding portion not part of the present invention;
Fig. 15 is a view showing a state in which the holding portion engages with the sliding tube not part of the present invention;
Fig. 16 is a view showing the configuration of a cleaning/disinfecting tube of
   the invention;
Fig. 17 is a view showing the configuration of a cleaning/disinfecting tube not part of the invention;
Fig. 18 is a cross-sectional view of a mouthpiece of an endoscope not part of the invention;
Fig. 19 is a cross-sectional view of a cleaning/disinfecting tube not part of the invention;
Fig. 20 is a view showing a state in which the cleaning/disinfecting tube not part of the invention is connected to the mouthpiece and a liquid is supplied;
Fig. 21 is a view showing a state in which the cleaning/disinfecting tube not part of the invention is connected to the mouthpiece and the supply of the liquid is terminated;
Fig. 22 is a cross-sectional view showing the configuration of a holding portion not part of the invention;
Fig. 23 is a plan view of a ring portion not part of the invention;
Fig. 24 is a cross-sectional view taken along the line XXIV-XXIV in Fig. 23;
Fig. 25 is a cross-sectional view of a mouthpiece of an endoscope in a seventh not part of the invention;
Fig. 26 is a cross-sectional view of a cleaning/disinfecting tube of the not part of the invention;
Fig. 27 is a cross-sectional view taken along the line XXVII-XXVII in Fig. 26;
Fig. 28 is a view for describing how to connect the cleaning/disinfecting tube not part of the invention;
Fig. 29 is a view showing a state in which the cleaning/disinfecting tube not part of the invention is connected to the mouthpiece and the supply of a liquid is terminated;
Fig. 30 is a view showing a state in which the cleaning/disinfecting tube of the not part of the invention is connected to the mouthpiece and the liquid is supplied;
Fig. 31 is a view showing a hole portion in a distal end portion of an ultrasound probe not part of the invention;
Fig. 32 is a view showing a cleaning/disinfecting tube not part of the invention; and
Fig. 33 is a view for describing an example of how to connect an endoscope to a cleaning/disinfecting tube of related art.

### Best Mode for Carrying Out the Invention

Preferred forms of an endoscope cleaning/disinfecting apparatus according to the present invention will be described with reference to the drawings. In the drawings used in the following description, the scale of each component is changed to show the component at a recognizable size on the drawings. The present invention is not limited only to the number of components, the shapes thereof, the size ratios thereof, and the relative positional relationships therebetween described in the drawings.

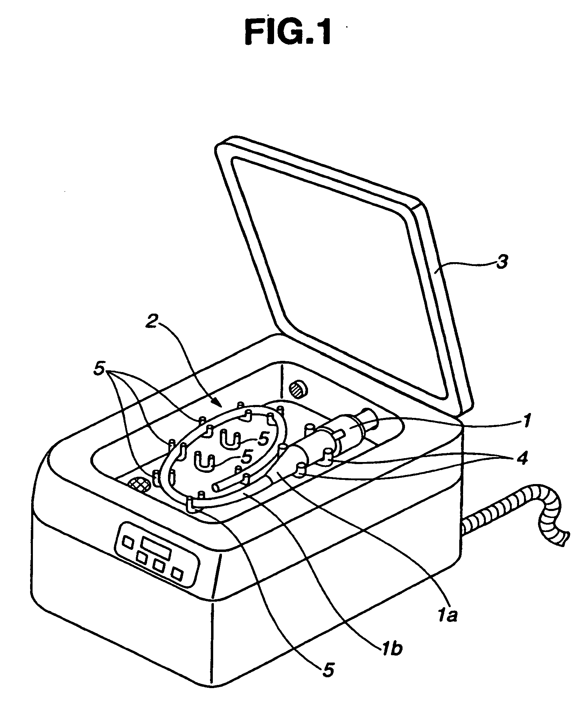

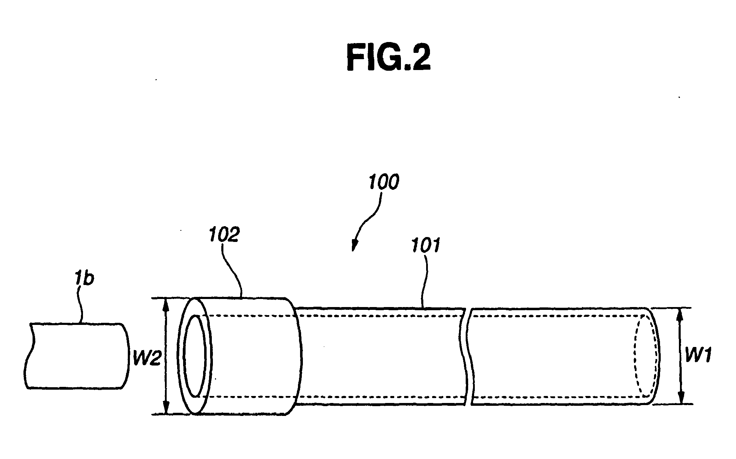

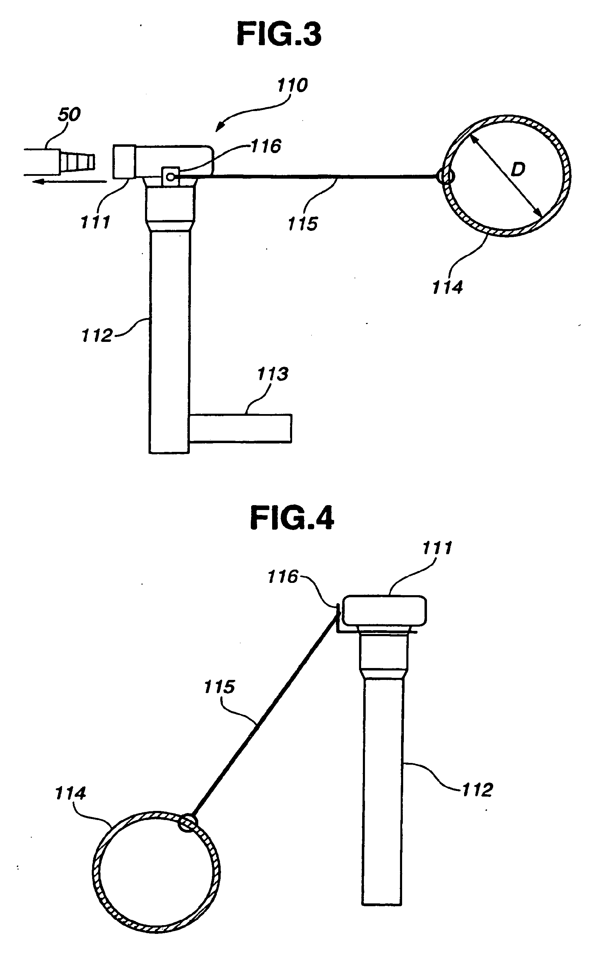

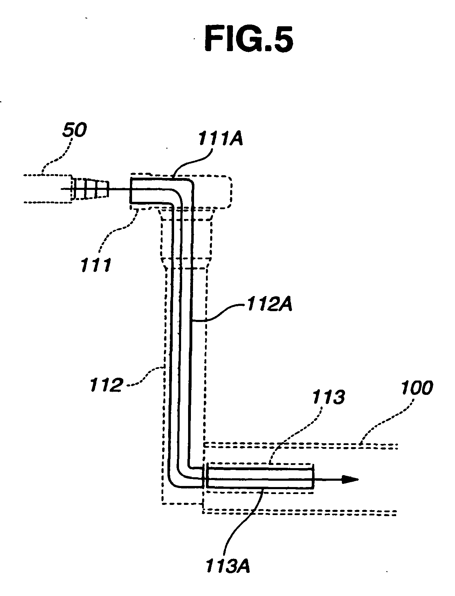

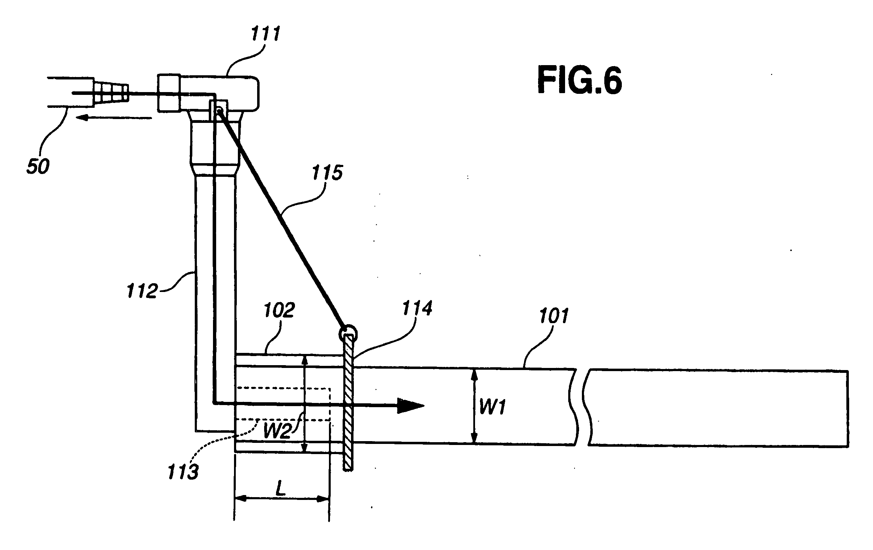

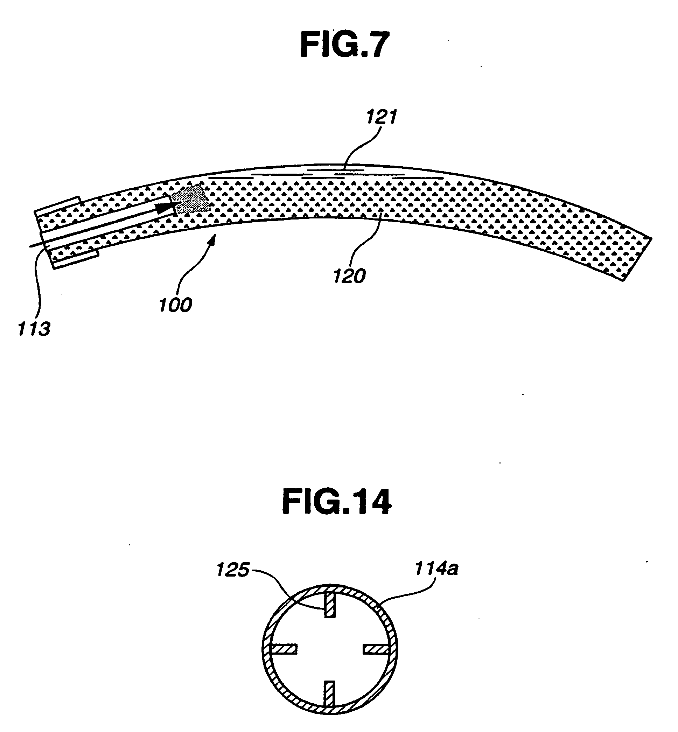

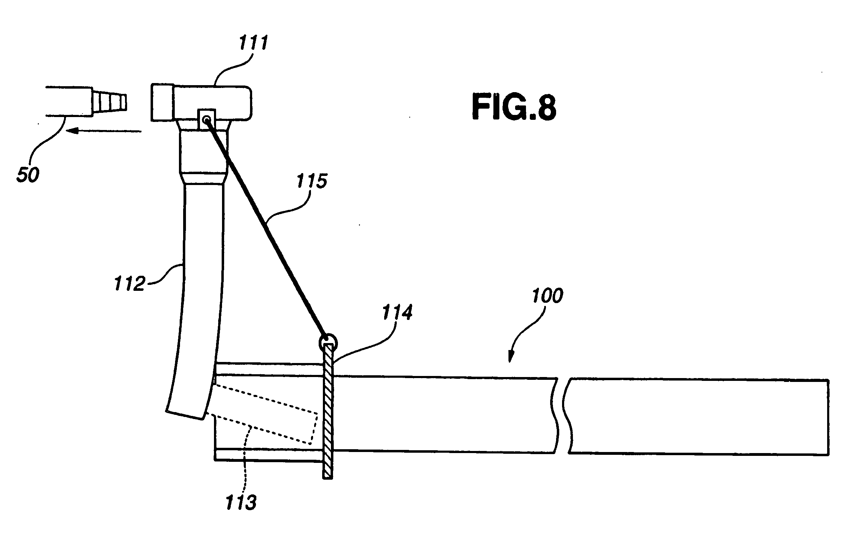

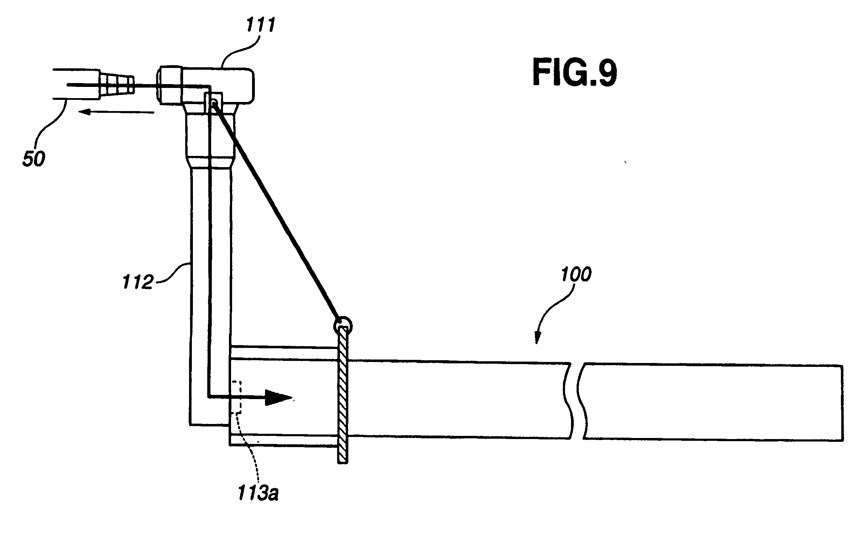

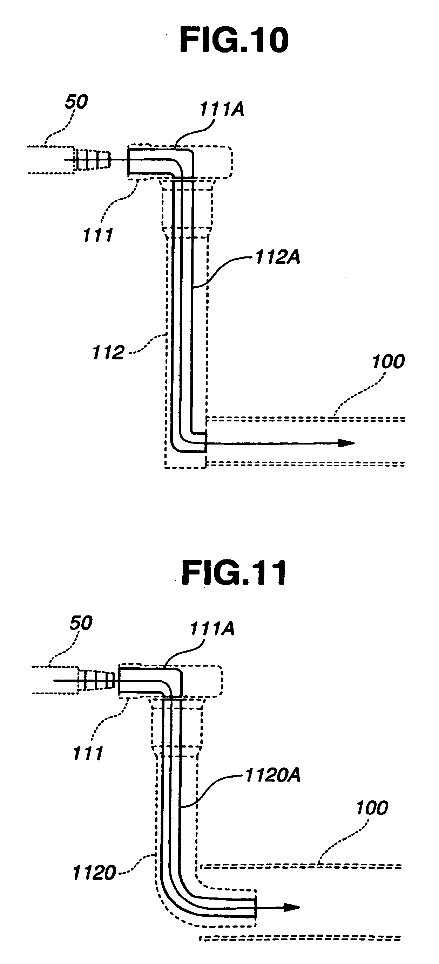

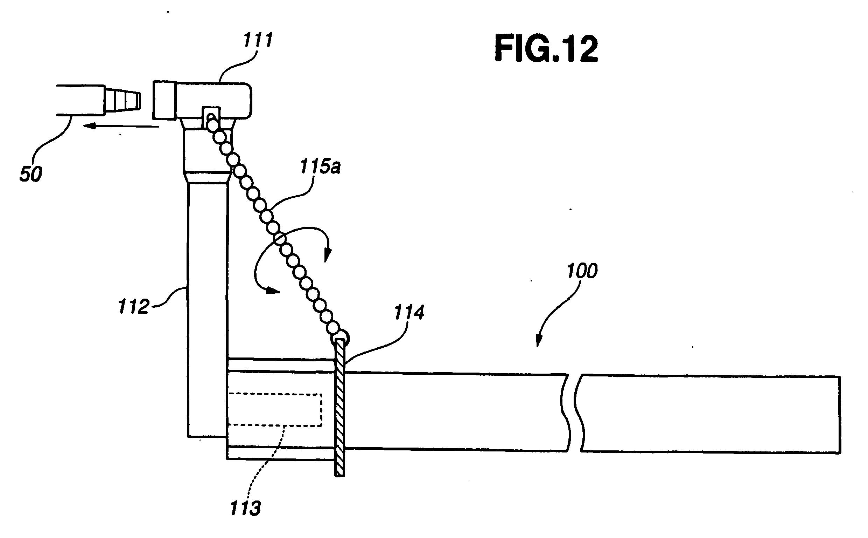

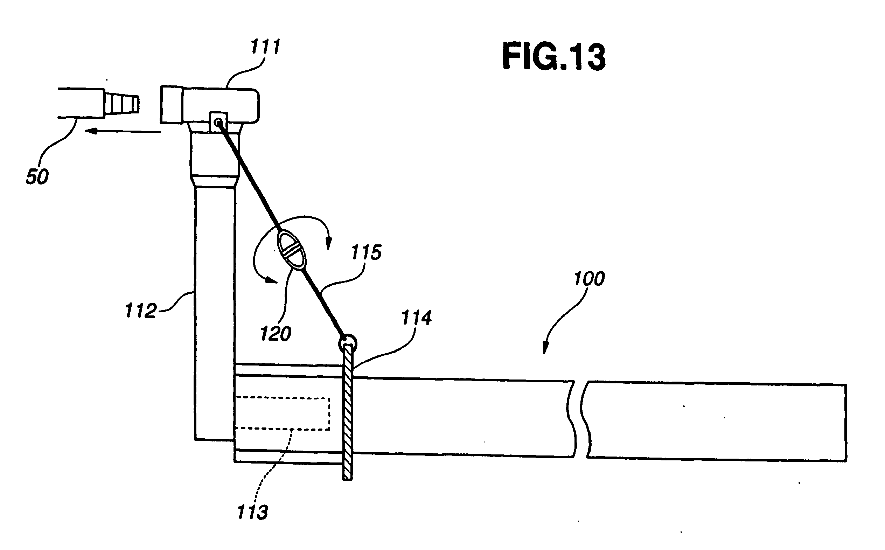

Fig. 1 is an exterior view showing the exterior of an endoscope cleaning/disinfecting cleaning/disinfecting apparatus. Fig. 2 is an exterior view showing the exterior of a sliding tube. Fig. 3 is a first view showing the configuration of a cleaning/disinfecting tube. Fig. 4 is a second view showing the cleaning/disinfecting tube. Fig. 5 is a cross-sectional view showing hollow paths in the cleaning/disinfecting tube. Fig. 6 is a view for describing the connection of the cleaning/disinfecting tube to the sliding tube. Fig. 7 is a view showing a state in which an air accumulation is created in the sliding tube. Fig. 8 is a view showing a state in which a connecting tube is deformed. Fig. 9 is a view showing a variation of a tube connector section. Fig. 10 is a view showing another variation of the tube connector section. Fig. 11 is a view showing another variation of the tube connector section. Fig. 12 is a view showing a variation of a connecting portion. Fig. 13 is a view showing another variation of the connecting portion.

The endoscope cleaning/disinfecting apparatus shown in Fig. 1 is an apparatus for cleaning and disinfecting an endoscope 1, attachments to the endoscope 1, and other objects to be cleaned in a cleaning tank 2. Cleaning and disinfecting the endoscope 1 in the endoscope cleaning/disinfecting apparatus are performed by using predetermined types of chemical solution, water, and other liquids.

A schematic configuration of the endoscope 1 will first be described. The endoscope 1 is primarily formed of a control section 1a, which is a portion used by an operator to operate the endoscope 1, and an insertion section 1b, which is a portion that can be introduced into a living body. Although not shown, one or more distal end opening portions are provided in a distal end portion of the insertion section 1b. On the other hand, the control section 1a has an opening portion that communicates with the distal end opening portions via a channel.

The endoscope 1 is designed in such a way that treatment instruments, such as puncture needles and forceps, can be introduced into a living body through the distal end opening portions of the insertion section 1b, for example, by inserting the treatment instruments through the opening portion of the control section 1a into the channel. The endoscope 1 can also, for example, perform a sucking action in the living body and deliver a liquid into the living body by transporting a fluid between the opening portion of the control section 1a and the distal end opening portions of the insertion section 1b, which communicate with each other via the channel. Since the configuration of the endoscope 1 is known, no description of the details thereof will be made.

As an example of how to use the endoscope 1, it is assumed in the present embodiment that a sliding tube 100 shown in Fig. 2 is used when the insertion section 1b of the endoscope 1 is introduced into a living body. The sliding tube 100 has a substantially cylindrical shape having an opening portion at both ends. The sliding tube 100 has a grasping portion 102 provided at one end and an insertion portion 101 provided at the other end and inserted into the living body. The outer diameter W2 of the grasping portion 102 is greater than the outer diameter W1 of the insertion portion 101.

A schematic configuration of the endoscope cleaning/disinfecting apparatus will next be described. The endoscope cleaning/disinfecting apparatus includes the cleaning tank 2 capable of accommodating the endoscope 1 therein and a lid 3 provided on the upper side of the cleaning tank 2.

Control section holding members 4 for holding the control section 1a of the endoscope 1 and insertion section holding members 5 for holding the insertion section 1b are disposed in the cleaning tank 2. Held by the control section holding members 4 and the insertion section holding members 5, the endoscope 1 is accommodated in the cleaning tank 2 in a predetermined position in a predetermined posture. The endoscope cleaning/disinfecting apparatus may alternatively be configured in such a way that the cleaning tank 2 can accommodate a plurality of endoscopes 1.

Although not shown, the cleaning tank 2 has a supply port through which a chemical solution or other liquid is supplied into the cleaning tank 2 and a discharge port through which the liquid in the cleaning tank 2 is discharged. Although not shown in Fig. 1, the cleaning tank 2 further has one or more liquid delivery connectors 50 through which a chemical solution or other liquid is supplied into the cleaning tank 2. The liquid delivery connector 50 can be connected to a cleaning/disinfecting tube 110, which will be described later.

The cleaning/disinfecting tube 110, which will be described later in detail, allows the liquid delivery connector 50 to communicate with the opening portion of the object to be cleaned accommodated in the cleaning tank 2. In the endoscope cleaning/disinfecting apparatus, the liquid supplied through the liquid delivery connector 50 via the cleaning/disinfecting tube 110 can be delivered through the opening portion of the object to be cleaned.

An inner surface of the sliding tube 100 can, for example, be cleaned and disinfected, for example, by using the cleaning/disinfecting tube 110 to connect the liquid delivery connector 50 to the opening portion at one end of the sliding tube 100 so that they communicate with each other. Similarly, the channel in the endoscope 1 can, for example, be cleaned and disinfected, for example, by using the cleaning/disinfecting tube 110 to connect the liquid delivery connector 50 to the opening portion of the control section 1a of the endoscope 1 so that they communicate with each other.

It is assumed in the present embodiment described below that the sliding tube 100 is an object to be cleaned and the cleaning/disinfecting tube 110 allows the liquid delivery connector 50 provided in the endoscope cleaning/disinfecting apparatus to communicate with the opening portion provided at one end of the sliding tube 100.

The cleaning/disinfecting tube 110 of the present embodiment includes a connector section 111 connectable to the liquid delivery connector 50 and a tube connector section 113 connectable to the opening portion of the sliding tube 100, as shown in Fig. 3. The connector section 111 and the tube connector section 113 are connected to each other via a connecting tube 112.

The connector section 111 has a hollow path 111A, which is connectable to the liquid delivery connector 50 in a watertight manner, as shown in Fig. 5. The tube connector section 113 has an outer shape that can fit into the opening portion at one end of the sliding tube 100 with a gap created therebetween. The tube connector section 113 has a hollow path 113A through which a liquid is delivered into the sliding tube 100 through the opening portion thereof after the tube connector section 113 fits into the opening portion. The hollow path 111A and the hollow path 113A communicate with each other through a hollow path 112A provided in the connecting tube 112.

The configuration described above allows the liquid supplied through the liquid delivery connector 50 to pass through the hollow path 111A, the hollow path 112A, and the hollow path 113A and be delivered through the opening portion of the sliding tube 100.

The cleaning/disinfecting tube 110 further includes a holding portion 114. The holding portion 114 is connected to a latching portion 116 provided at the connector section 111 via a flexible connecting portion 115. The holding portion 114 can engage with an outer circumferential portion of the sliding tube 100.

The configuration in which the holding portion 114 engages with the sliding tube 100 is not particularly limited to a specific one. As an example, the holding portion 114 fits on the outer circumferential portion of the sliding tube 100.

Specifically, the holding portion 114 has a through hole into which the insertion portion 101 of the sliding tube 100 can fit with a gap therebetween but into which the grasping portion 102 cannot fit. For example, when the through hole has a substantially circular shape having a diameter D, as shown in Fig. 3, the diameter D is greater than the outer diameter W1 of the insertion portion 101 but smaller than the outer diameter W2 of the grasping portion 102. As a result, when the sliding tube 100 is inserted, with the insertion portion 101 being a leading portion, into the through hole of the holding portion 114 until the grasping portion 102 comes into contact with the holding portion 114, the holding portion 114 engages with the sliding tube 100 and the relative positional relationship between them is fixed.

The connecting portion 115 is configured in such a way that after the holding portion 114 engages with the sliding tube 100, the tube connector section 113 can fit through the opening portion of the sliding tube 100 and the movement of the sliding tube 100 in a direction away from the tube connector section 113 is constrained, as shown in Fig. 6.

The movement of the sliding tube 100 in a direction away from the tube connector section 113 refers to an action in which the sliding tube 100 moves relative to and away from the tube connector section 113 substantially along a central axis of the sliding tube 100.

The configuration of the connecting portion 115 is not particularly limited to a specific one, but the connecting portion 115 is formed of a chain-shaped member . The connecting portion 115 may alternatively be a cord-shaped member. Further, the connecting portion 115 may be formed of a plurality of connecting portions 115.

When the thus configured cleaning/disinfecting tube 110 is used to connect the liquid delivery connector 50 to the opening portion of the sliding tube 100 in such a way that they communicate with each other, among the portions that form the cleaning/disinfecting tube 110, the holding portion 114 and part of the tube connector section 113 probably come into contact with the sliding tube 100, as shown in Fig. 6.

When the sliding tube 100 is immersed in a liquid and the liquid is supplied through the liquid delivery connector 50, the friction between the sliding tube 100 and the liquid delivered through the tube connector section 113 generates a force acting on the sliding tube 100 and oriented in a direction away from the tube connector section 113 substantially along the central axis of the sliding tube 100. Since the holding portion 114 constrains the movement of the sliding tube 100 in the direction away from the tube connector section 113, the tube connector section 113 will not disengage from the opening portion of the sliding tube 100.

At this point, since the tube connector section 113 fits into the opening portion of the sliding tube 100 with a gap therebetween and the sliding tube 100 tends to move away from the tube connector section 113, a gap is created between the sliding tube 100 and the tube connector section 113.

That is, when the liquid is supplied through the liquid delivery connector 50, among the portions of the cleaning/disinfecting tube 110 that probably come into contact with the sliding tube 100, the tube connector section 113 is spaced apart from the sliding tube 100. According to the present embodiment, supplying the liquid through the liquid delivery connector 50 therefore allows the liquid to flow between the tube connector section 113 and the sliding tube 100.

On the other hand, when the sliding tube 100 is immersed in the liquid but the supply of the liquid through the liquid delivery connector 50 is terminated, the force that moves the sliding tube 100 away from the tube connector section 113 does not act on the sliding tube 100. As a result, the position of the sliding tube 100 can freely be changed relative to that of the holding portion 114.

That is, when the supply of the liquid through the liquid delivery connector 50 is terminated, among the portions of the cleaning/disinfecting tube 110 that probably come into contact with the sliding tube 100, at least the holding portion 114 is spaced apart from the sliding tube 100. According to the present disclosure, stopping supplying the liquid through the liquid delivery connector 50 therefore allows the liquid to flow between the holding portion 114 and the sliding tube 100.

As described above, using the cleaning/disinfecting tube 110 of the present embodiment allows the entire sliding tube 100, which is an object to be cleaned, to be immersed in a chemical solution, water, and other liquids in a reliable manner.

More preferable forms of the tube connector section 113 will be described below.

For example, it is conceivable, as shown in Fig. 7, that an air accumulation 121 is possibly created in the sliding tube 100 depending on the posture and the shape of the sliding tube 100 in the cleaning tank 2. In this case, a length L (shown in Fig. 6) of the tube connector section 113 is preferably short enough not to reach the air accumulation 121.

When the length L of the tube connector section 113 is thus short enough not to reach the air accumulation 121, the air accumulation 121 can be removed from the sliding tube 100 by making use of the liquid delivered through the tube connector section 113. As a result, the entire sliding tube 100 can be immersed in the liquid in a more reliable manner.

Further, for example, when the connecting tube 112 is elastically deformed, it is conceivable that the tube connector section 113 possibly falls off the opening portion of the sliding tube 100, as shown in Fig. 8. The length L and the modulus of elasticity of the tube connector section 113 are preferably determined in such a way that the tube connector section 113 will not fall off even when the tube connector section 113 obliquely fits into the sliding tube 100 as described above. Specifically, it is preferable that the length L of the tube connector section 113 is greater than the inner diameter of the opening portion of the sliding tube 100 and the modulus of elasticity of the tube connector section 113 is higher than that of the connecting tube 112.

On the other hand, when the connecting tube 112 used in the endoscope cleaning/disinfecting apparatus is not substantially deformed, the length L of a tube connector section 113a can be determined irrespective of the diameter of the opening portion of the sliding tube 100, as shown in Fig. 9. The tube connector section may alternatively not protrude into the sliding tube 100, as shown in Fig. 10.

The above disclosure has been illustrated and described with reference to the configuration in which the tube connector section extends sideward from a distal end portion of the connecting tube, but the tube connector section may extend from the distal end portion of the connecting tube along the central axis thereof.

For example, a tube connector section 1120 may be integrated with a connecting tube and has a hollow path 1120A therein, as shown in Fig. 11. Further, the tube connector section 1120 may be made of a flexible material. In this case, a distal end portion can be bent, and the bent distal end portion can be inserted into the sliding tube 100, as shown in Fig. 11.

More preferable forms of the connecting portion 115 in the present embodiment described above will be described below.

For example, a connecting portion 115a is preferably formed of a ball-chain member at least part of which is rotatable in a twisted direction, as shown in Fig. 12. The configuration accurately constrains the distance between the sliding tube 100 and the tube connector section 113 because the length of the connecting portion 115a does not change even when the connecting portion 115a is twisted to allow the holding portion 114 to engage with the sliding tube 100.

The configuration in which at least part of the connecting portion is rotatable in the twisted direction does not necessary use the ball-chain member, but the connecting portion 115 may have a swivel member 120, for example, as shown in Fig. 13.

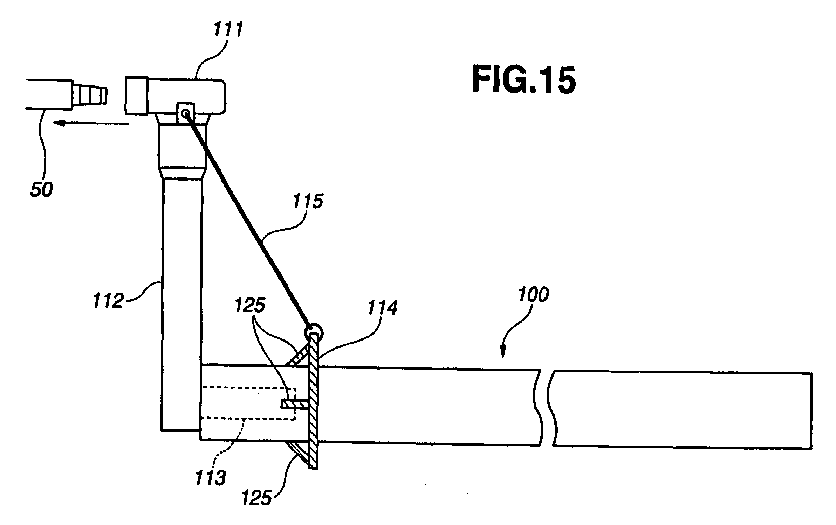

A second cleaning/disinfecting tube not part of the present invention will be described below. Fig. 14 is a view showing the configuration of a holding portion in the second embodiment. Fig. 15 is a view showing a state in which the holding portion engages with the sliding tube.

The second tube differs from the first tube in terms of the configuration of the holding portion. Only the difference between the first and second tubes will be described below. The same components as those in the first tube have the same reference characters, and no description of those components will be made as appropriate.

In the present embodiment, a holding portion 114a frictionally engages with an outer circumferential portion of the sliding tube 100. Specifically, the holding portion 114a has frictional engaging portions 125 extending from an inner circumferential surface of a through hole toward the center, as shown in Fig. 14.

When the sliding tube 100 is inserted into the through hole of the holding portion 114a, the frictional engaging portions 125 come into contact with the outer circumferential portion of the sliding tube 100 with the distal ends of the frictional engaging portions 125 inclined in the direction in which the sliding tube 100 is inserted, as shown in Fig. 15. In this state, when the sliding tube 100 is forced to move relative to the holding portion 114a in the opposite direction to the insertion direction, the friction between the sliding tube 100 and the frictional engaging portions 125 resists the movement.

Using a cleaning/disinfecting tube 110 including the holding portion 114a described above, which frictionally engages with the outer circumferential portion of the sliding tube 100, allows a sliding tube 100 with no step around the outer circumferential portion to be cleaned and disinfected.

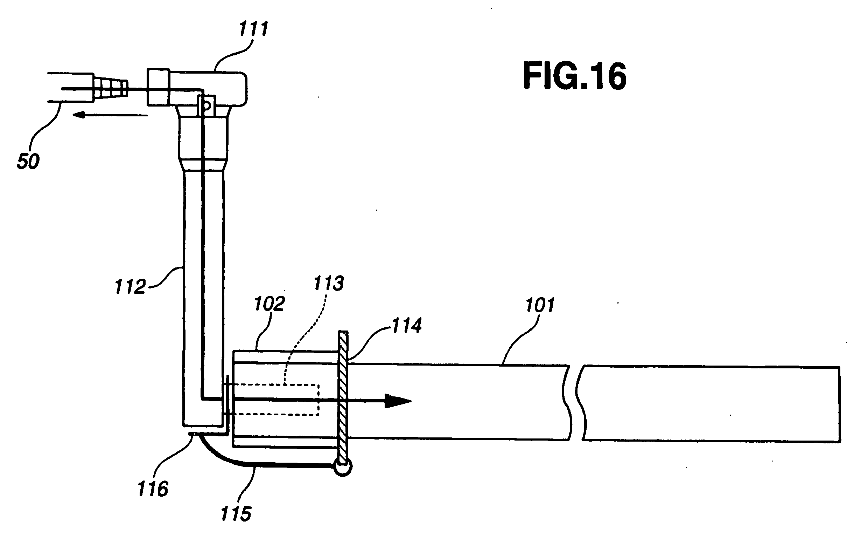

A tube according to the present invention will be described below. Fig. 16 is a view showing the configuration of a cleaning/disinfecting tube according to the invention.

The tube according to the invention differs from the first tube in terms of the configuration of the latching portion. Only the difference between the first tube and the tube according to the invention will be described below. The same components as those in the first tube have the same reference characters, and no description of those components will be made as appropriate.

In the present embodiment, the latching portion 116 is disposed at a base portion of the tube connector section 113, as shown in Fig. 16. The configuration described above also allows the movement of the sliding tube 100 in a direction away from the tube connector section 113 to be constrained, whereby the sliding tube 100 can be cleaned and disinfected, as in the first tube.

Further, providing the latching portion 116 at the tube connector section 113 prevents the linear distance between the latching portion 116 and the tube connector section 113 from changing even when the connecting tube 112 is deformed. As a result, the distance by which the sliding tube 100 is spaced apart from the tube connector section 113 can be maintained irrespective of any change in the shape of the connecting tube 112, whereby the entire sliding tube 100 can be immersed in a liquid in a more reliable manner. The length of the connecting portion 115 is preferably adjustable.

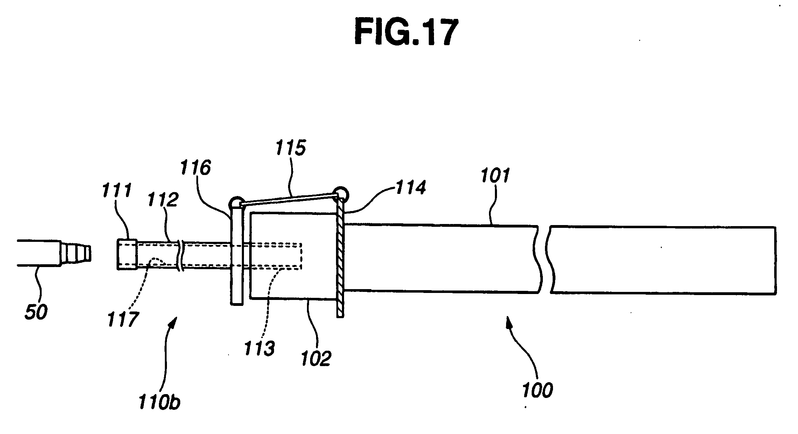

A fourth tube not part of the present invention will be described below. Fig. 17 is a view showing the configuration of a cleaning/disinfecting tube of the fourth tube.

The fourth tube differs from the tube according to the invention in terms of the shape of the cleaning/disinfecting tube. Only the difference between the tube according to the invention and fourth tube will be described below. The same components as those in the tube according to the invention have the same reference characters, and no description of those components will be made as appropriate.

The path through which a liquid is delivered in the cleaning/disinfecting tube 110 of the third embodiment described above is a bent path formed of the hollow paths 111A, 112A, and 113A. A cleaning/disinfecting tube 110b of the present embodiment has the connector section 111, the connecting tube 112, and the tube connector section 113 disposed substantially along a straight line, as shown in Fig. 17. The cleaning/disinfecting tube 110b has a substantially linear hollow path 117 that passes through the connector section 111, the connecting tube 112, and the tube connector section 113.

The configuration described above, of course, also allows the sliding tube 100 to be cleaned and disinfected, as in the tube according to the invention.

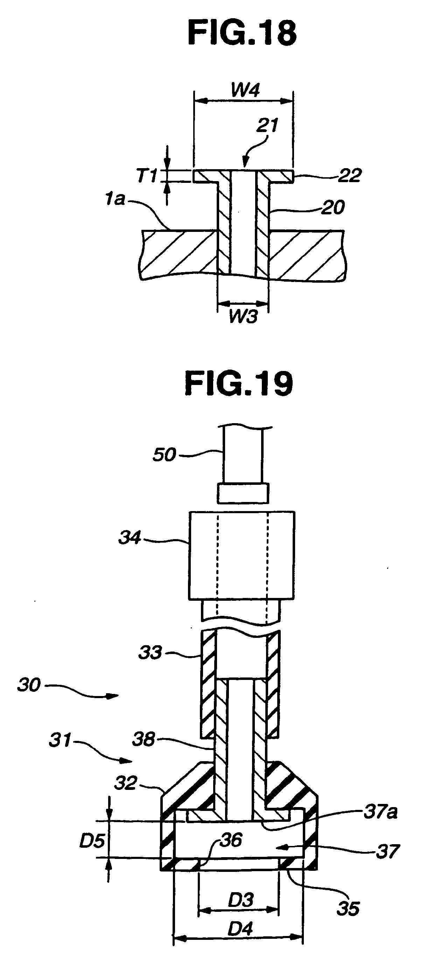

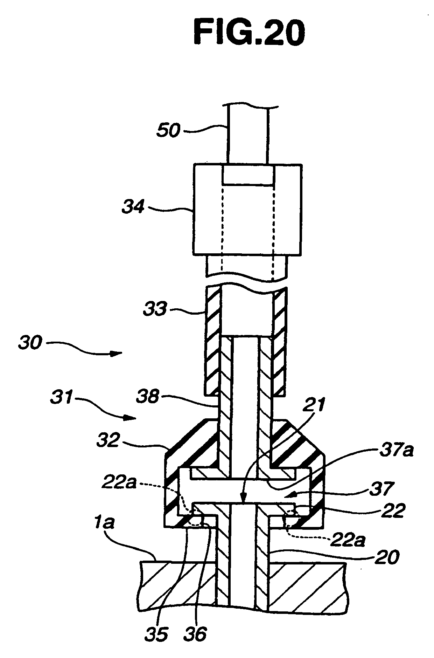

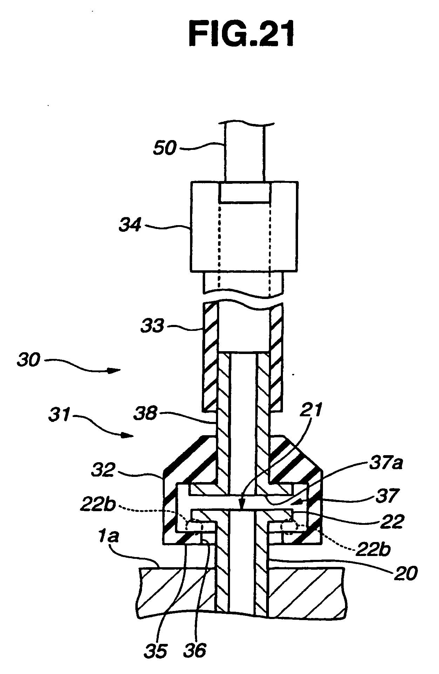

A fifth tube not part of the present invention will be described below. Fig. 18 is a cross-sectional view of a mouthpiece of an endoscope. Fig. 19 is a cross-sectional view of a cleaning/disinfecting tube of the fifth embodiment. Fig. 20 is a view showing a state in which the cleaning/disinfecting tube is connected to the mouthpiece and a liquid is supplied. Fig. 21 is a view showing a state in which the cleaning/disinfecting tube is connected to the mouthpiece and the supply of the liquid is terminated.

The fifth tube differs from the first to fourth tubes in that the object to be cleaned is the endoscope 1. The control section 1a of the endoscope 1 of the present embodiment includes a mouthpiece 20 having an opening portion 21 through which a treatment instrument or any other component is inserted. A cleaning/disinfecting tube 30 of the present embodiment allows the liquid delivery connector 50 provided in the cleaning tank 2 of the endoscope cleaning/disinfecting apparatus to communicate with the opening portion 21 of the mouthpiece 20 of the endoscope 1, which is the object to be cleaned.

The mouthpiece 20 is a substantially tubular member provided at the control section 1a in a protruding manner. The opening portion 21 of the mouthpiece 20 communicates with the distal end openings (not shown) provided in the insertion section 1b of the endoscope 1. A flange portion 22 extending outward in the circumferential direction is provided at a distal end portion of the mouthpiece 20.

As shown in Fig. 18, the mouthpiece 20 has, for example, a substantially cylindrical shape having a diameter W3, and the flange portion 22 having a diameter W4 greater than the diameter W3 is formed at the distal end portion of the substantially cylindrical portion.

The cleaning/disinfecting tube 30 includes a tube connector section 31, a connecting tube 33, and a connector section 34, as shown in Fig. 19.

The connector section 34 can be connected to the liquid delivery connector 50 of the endoscope cleaning/disinfecting apparatus in a watertight manner. Although not shown, the connector section 34 includes a lock mechanism and a release mechanism for locking and releasing the connection between the liquid delivery connector 50 and the connector section 34.

The connector section 34 is connected to the proximal end of the connecting tube 33 having a hollow path. The tube connector section 31 is connected to the distal end of the connecting tube 33.

The tube connector section 31 is formed of a tubular portion 38 and a holding portion 32. The tubular portion 38 is a substantially tubular member. A proximal end portion of the tubular portion 38 is inserted into the distal end of the connecting tube 33 and fixed there.

The holding portion 32 is a member that is disposed at a distal end portion of the tubular portion 38 and can engage with the mouthpiece 20 of the endoscope 1 with a gap therebetween. In the present disclosure, the holding portion 32 is made of rubber, a silicon resin, or any other suitable elastic material.

Specifically, a mouthpiece insertion port 36, which is a through hole, is formed at a distal end portion of the holding portion 32, and a space 37 capable of accommodating the flange portion 22 of the mouthpiece 20 inserted through the mouthpiece insertion port 36 is formed on the proximal end side of the mouthpiece insertion port 36. The space 37 communicates with the connecting tube 33 via the tubular portion 38.

More specifically, the mouthpiece insertion port 36 formed at the distal end portion of the holding portion 32 is a through hole having a substantially circular shape having a diameter D3 smaller than the diameter W4 of the flange portion 22 of the mouthpiece 20 but greater than the diameter W3 of the mouthpiece 20. The space 37 has a substantially cylindrical shape substantially coaxial with the mouthpiece insertion port 36 and has a diameter greater than the diameter W4 of the flange portion 22 of the mouthpiece 20. The depth D5 of the space 37 is greater than the thickness T1 of the flange portion 22.

Since the thus configured holding portion 32 is made of an elastic material, the holding portion 32 is deformed when the mouthpiece 20 is pressed against the mouthpiece insertion port 36 by a predetermined force, whereby the space 37 can accommodate the flange portion 22 of the mouthpiece 20, as shown in Fig. 20.

In other words, when the flange portion 22 is accommodated in the space 37 of the holding portion 32, an engaging portion 35, which has the mouthpiece insertion port 36, the diameter D3 of which is smaller than the diameter W4 of the flange portion 22, engages with an outer circumferential portion of the mouthpiece 20. In this state, the engaging portion 35 constrains the movement of the mouthpiece 20 in a direction away from the tube connector section 31. The movement of the mouthpiece 20 in a direction away from the tube connector section 31 refers in the present embodiment to an action in which the mouthpiece 20 moves relative to and away from the tube connector section 31 substantially along a central axis of the mouthpiece 20.

When the holding portion 32 engages with the mouthpiece 20, the opening portion 21 of the mouthpiece 20 communicates with the liquid delivery connector 50 via the space 37, the tubular portion 38, the connecting tube 33, and the connector section 34. In the state in which the holding portion 32 engages with the mouthpiece 20 as shown in Fig. 20, the holding portion 32 is deformed when a force having at least a predetermined magnitude is applied to the mouthpiece 20 in a direction away from the holding portion 32, whereby the flange portion 22 can be removed from the space 37 and the holding portion 32 can disengage from the mouthpiece 20.

When the thus configured cleaning/disinfecting tube 30 is used to connect the liquid delivery connector 50 to the opening portion 21 of the mouthpiece 20 in such a way that they communicate with each other, among the portions that form the cleaning/disinfecting tube 30, the engaging portion 35 and a bottom portion 37a of the space 37 probably come into contact with the mouthpiece 20, as shown in Fig. 20.

For example, the endoscope 1, that is, the mouthpiece 20 is immersed in a liquid, and the liquid is supplied through the liquid delivery connector 50, the pressure of the liquid in the space 37 generates a force acting on the tube connector section 31 and oriented in a direction away from the mouthpiece 20. The engaging portion 35 of the holding portion 32 therefore comes into contact with the flange portion 22, as shown in Fig. 20 (22a in Fig. 20).

The bottom portion 37a of the space 37 is now immersed in the liquid because the bottom portion 37a is spaced apart from the mouthpiece 20. Further, since the liquid supplied through the liquid delivery connector 50 is forcibly delivered through the opening portion 21 into the channel in the endoscope 1, the entire channel is filled with the liquid.

On the other hand, when the endoscope 1, that is, the mouthpiece 20 is immersed in the liquid, and the supply of the liquid through the liquid delivery connector 50 is terminated, the pressure of the liquid in the space 37 becomes equal to that in the environment, whereby the mouthpiece 20 can freely move in the space 37. As a result, a gap is created between the flange portion 22 and the engaging portion 35 (22b in Fig. 21), and the entire flange portion 22 is immersed in the liquid, as shown in Fig. 21.

As described above, when the cleaning/disinfecting tube 30 of the present embodiment is used to connect the liquid delivery connector 50 to the opening portion 21 of the mouthpiece 20 of the endoscope 1 in such a way that they communicate with each other, the liquid can be supplied into the channel, which communicates with the mouthpiece 20, and the entire mouthpiece 20, to which the tube connector section 31 is connected, can be immersed in the liquid in a reliable manner.

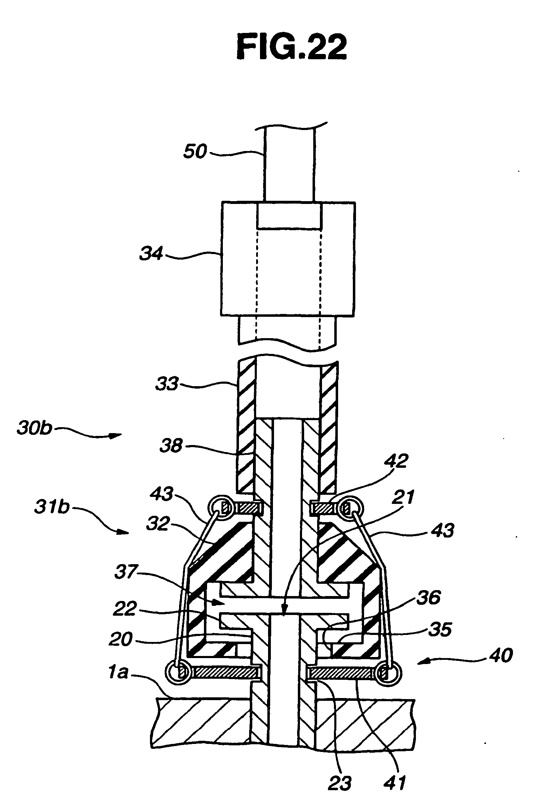

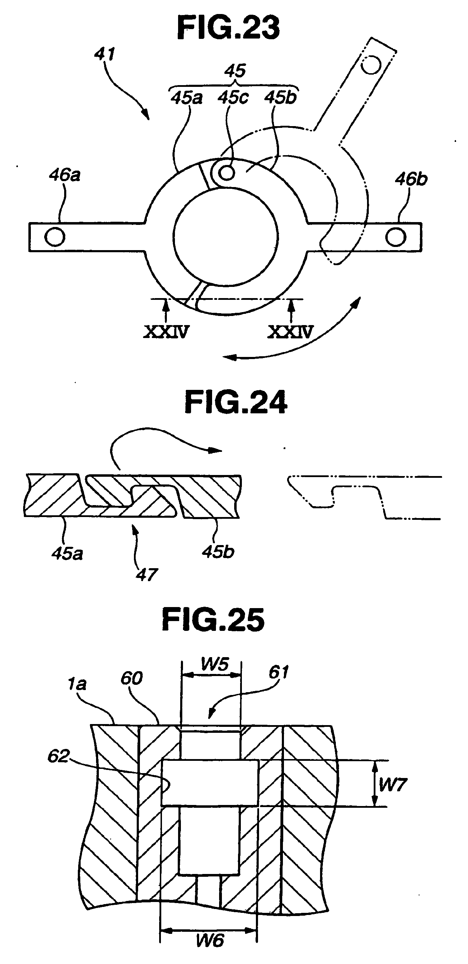

A sixth tube not part of the present invention will be described below. Fig. 22 is a cross-sectional view showing the configuration of a holding portion. Fig. 23 is a plan view of a ring portion. Fig. 24 is a cross-sectional view taken along the line XXIV-XXIV in Fig. 23.

A cleaning/disinfecting tube 30b differs from the fifth tube in terms of the configuration of a tube connector section 31b. Only the difference between the fifth and sixth tubes will be described below. The same components as those in the first tube have the same reference characters, and no description of those components will be made as appropriate.

The tube connector section 31b in the sixth tube differs from that in the fifth tube in that a second holding portion 40 is further provided. The second holding portion 40 engages with the mouthpiece 20 of the endoscope 1 with a gap therebetween and constrains the movement of the mouthpiece 20 in a direction away from the tube connector section 31b.

As shown in Fig. 22, the second holding portion 40 includes an engaging portion 41 that engages with the outer circumferential portion of the mouthpiece 20, a latching portion 42 provided at the tubular portion 38, and connecting portions 43 that connect the engaging portion 41 to the tubular portion 38. The engaging portion 41 can engage the mouthpiece 20 and disengage therefrom.

In the present tube, the engaging portion 41 includes, for example, a ring portion 45 that engages with a groove portion 23 formed around the outer circumferential portion of the mouthpiece 20 and a plurality of arm portions 46a and 46b extending from the ring portion 45 outward in the radial direction, as shown in Fig. 23.

The ring portion 45 is divided into semicircular portions 45a and 45b. One end portion of the semicircular portion 45a is connected to one end portion of the semicircular portion 45b with a pin 45c so that the semicircular portions 45a and 45b freely pivot around the pin 45c with respect to each other, as indicated by the chain double-dashed line in Fig. 23. Other end portions of the semicircular portions 45a and 45b form a lock mechanism 47 for maintaining the other end portions connected to each other, for example, as shown in Fig. 24.

After the lock mechanism 47 is released and the groove portion 23 of the mouthpiece 20 is placed inside the semicircular portions 45a and 45b, connecting the semicircular portions 45a and 45b by using the lock mechanism 47 allows the ring portion 45 to engage with the groove portion 23 of the mouthpiece 20. The arm portions 46a and 46b can be connected to the connecting portions 43, which will be described later.

The latching portion 41 and the connecting portions 43 are provided to constrain the distance by which the engaging portion 41 is spaced apart from the tube connector section 31b. Each of the connecting portions 43 is formed of a flexible member having a predetermined length and connects the latching portion 42 fixed to the tubular portion 38 to the engaging portion 41 described above.

In the present tube, each of the connecting portions 43 is formed of a chain-shaped member. Each of the connecting portions 43 may alternatively be a cord-shaped member. The latching portion 42 may not be disposed at the tubular portion 38 but may alternatively be disposed at the holding portion 32, the connecting tube 33, or the connector section 34. Three or more connecting portions 43 may be provided.

The length of each of the connecting portions 43 is determined in such a way that the flange portion 22 does not come into contact with the engaging portion 35 of the holding portion 32 when the flange portion 22 of the mouthpiece 20 is accommodated in the space 37 of the holding portion 32 and a predetermined force is applied to the mouthpiece 20 in a direction in which the mouthpiece 20 is moved away from the tube connector section 31b.

In other words, the second holding portion 40 constrains the movement of the mouthpiece 20 in such a way that a gap is created between the mouthpiece 20 and the holding portion 32 when the mouthpiece 20 is moved relative to and away from the tube connector section 31b.

When the thus configured cleaning/disinfecting tube 30b is used to connect the liquid delivery connector 50 to the opening portion 21 of the mouthpiece 20 so that they communicate with each other, and the liquid is supplied through the liquid delivery connector 50, as shown in Fig. 22, the pressure of the liquid in the space 37 generates a force acting on the tube connector section 31b and oriented in a direction away from the mouthpiece 20.

At this point, since the second holding portion 40 that engages with the mouthpiece 20 constraints the distance between the tube connector section 31b and the mouthpiece 20, a gap is created between the mouthpiece 20 and the holding portion 32.

As a result, the liquid supplied through the liquid delivery connector 50 is not only delivered through the opening portion 21 of the mouthpiece 20 into the channel in the endoscope 1 but also delivered through the gap between the mouthpiece 20 and the holding portion 32 and discharged out of the space 37. That is, the liquid can flow around the entire mouthpiece 20, whereby the entire mouthpiece 20 can be immersed in the liquid in a more reliable manner.

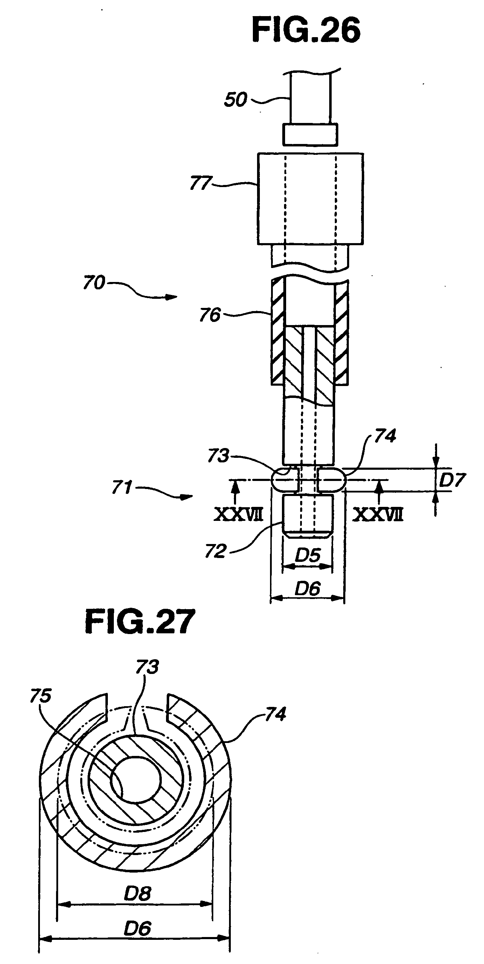

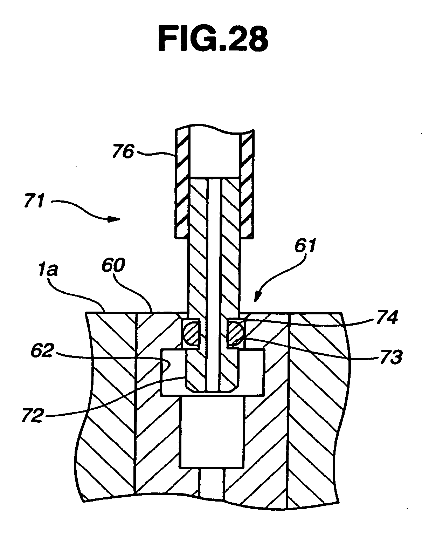

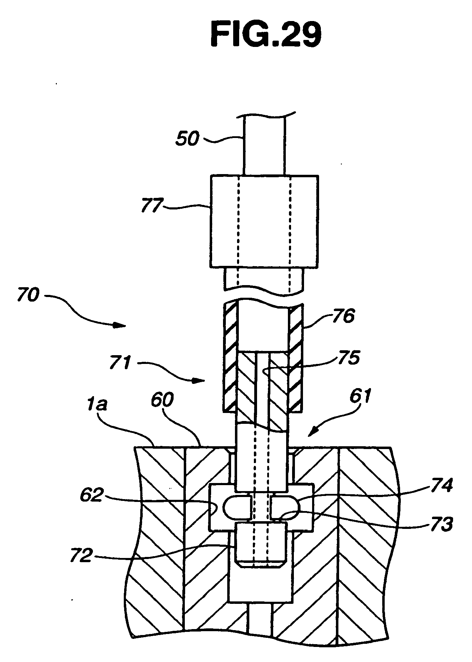

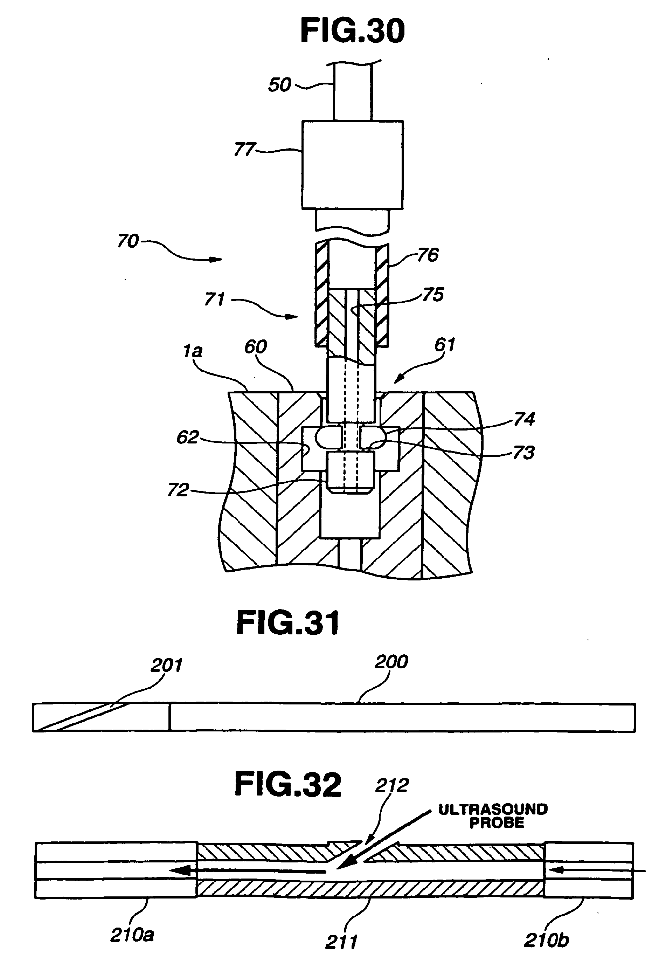

A seventh tube not part of the present invention will be described below. Fig. 25 is a cross-sectional view of a mouthpiece of an endoscope. Fig. 26 is a cross-sectional view of a cleaning/disinfecting tube of the seventh embodiment. Fig. 27 is a cross-sectional view taken along the line XXVII-XXVII in Fig. 26. Fig. 28 is a view for describing how to connect the cleaning/disinfecting tube to the mouthpiece. Fig. 29 is a view showing a state in which the cleaning/disinfecting tube is connected to the mouthpiece and the supply of a liquid is terminated. Fig. 30 is a view showing a state in which the cleaning/disinfecting tube is connected to the mouthpiece and the liquid is supplied.

In the seventh tube, the object to be cleaned is the endoscope 1. The control section 1a of the endoscope 1 of the present embodiment has a mouthpiece 60 having an opening portion 61 that communicates with the distal end openings in the insertion section 1b, as shown in Fig. 25. A cleaning/disinfecting tube 70 of the present embodiment is provided to allow the liquid delivery connector 50 provided in the cleaning tank 2 of the endoscope cleaning/disinfecting apparatus to communicate with the opening portion 61 of the mouthpiece 60 of the endoscope 1, which is the object to be cleaned.

The mouthpiece 60 is a substantially tubular member provided in the control section 1a, and the opening portion 61 having a diameter W5 is formed in the mouthpiece 60. A groove portion 62 having a diameter W6 greater than the diameter W5 is formed in an inner circumferential surface portion of the mouthpiece 60 along the circumferential direction. The width of the groove portion 62 in the direction along the central axis of the mouthpiece 60 is W7.

As shown in Fig. 26, the cleaning/disinfecting tube 70 includes a tube connector section 71, a connecting tube 76, and a connector section 77. The connector section 77 can be connected to the liquid delivery connector 50 of the endoscope cleaning/disinfecting apparatus in a watertight manner. Although not shown, the connector section 77 includes a lock mechanism and a release mechanism for locking and releasing the connection between the liquid delivery connector 50 and the connector section 77. The connector section 77 is connected to the proximal end of the connecting tube 76 having a hollow path. The tube connector section 71 is connected to the distal end of the connecting tube 76.

The tube connector section 71 is formed of a tubular portion 72 and a holding portion 74. The tubular portion 72 is a substantially cylindrical member having a diameter D5 smaller than the diameter W5 of the opening portion 61 of the mouthpiece 60. A proximal end portion of the tubular portion 72 is inserted into the distal end of the connecting tube 76 and fixed there. A groove portion 73 is formed in a side surface portion of the tubular portion 72 along the circumferential direction.

The holding portion 74 is what is called a C-ring-shaped member formed of a ring with a cut portion and fits on the groove portion 73 in the tubular portion 72 with a gap therebetween, as shown in Fig. 27. The diameter D6 of the holding portion 74, when no force is applied thereto, is greater than the diameter W5 of the opening portion 61 of the mouthpiece 60 but smaller than the diameter W6 of the groove portion 62 of the mouthpiece 60. The thickness D7 of the holding portion 74 is smaller than the width W7 of the groove portion 62 of the mouthpiece 60.

The holding portion 74 is made of a metal, a resin, or another suitable elastically deformable material, and can be elastically deformed and reduced to a diameter D8, which is smaller than the diameter W5 of the opening portion 61 of the mouthpiece 60, when an external force oriented inward in the radial direction is applied to the holding portion 74.

The shape of the holding portion 74 is not limited to the C-ring shape but may be any shape that can be elastically deformed by an external force so that the diameter D6 is changed to the diameter D8. For example, when the holding portion 74 is made of rubber or any other similar material, the holding portion 74 may have an O-ring shape because an external force can elastically deform the holding portion 74 from the diameter D6 to D8.

Since the holding portion 74 can be deformed to the diameter D8 smaller than the diameter W5, the holding portion 74 is inserted through the opening portion 61 of the mouthpiece 60 by pushing the tube connector section 71 into the opening portion 61, as shown in Fig. 28. The holding portion 74 can then be inserted into the groove portion 62 of the mouthpiece 60, as shown in Fig. 29, by further pushing the tube connector section 71 in the state shown in Fig. 28.

In the state shown in Fig. 29, the holding portion 74 is elastically deformed and enlarged back to the diameter D6 greater than the diameter W5 of the opening portion 61. As described above, the diameter D6 of the holding portion 74 at this point is smaller than the diameter W6 of the groove portion 62 of the mouthpiece 60.

As a result, when the tube connector section 71 is inserted until the holding portion 74 reaches the groove portion 62, the holding portion 74 engages with the groove portion 62 in an inner circumferential portion of the mouthpiece 60 with a gap therebetween, as shown in Fig. 29. At this point, the holding portion 74 constraints the movement of the mouthpiece 60 in a direction away from the tube connector section 71. The movement of the mouthpiece 60 in a direction away from the tube connector section 71 refers in the present embodiment to an action in which the mouthpiece 60 moves relative to and away from the tube connector section 71 substantially along a central axis of the mouthpiece 60.

When the holding portion 74 engages with the mouthpiece 60, the opening portion 61 of the mouthpiece 60 communicates with the liquid delivery connector 50 via the tubular portion 72, the connecting tube 76, and the connector section 77. In the state in which the holding portion 74 engages with the mouthpiece 60 as shown in Fig. 29, the holding portion 74 is elastically deformed and disengages from the mouthpiece 60 when a force having at least a predetermined magnitude is applied to the tube connector section 71 in a direction away from the mouthpiece 60, whereby the tube connector section 71 can be removed from the mouthpiece 60.

When the thus configured cleaning/disinfecting tube 70 is used to connect the liquid delivery connector 50 to the opening portion 61 of the mouthpiece 60 in such a way that they communicate with each other, and a liquid is supplied through the liquid delivery connector 50, the pressure of the liquid in the groove portion 62 generates a force acting on the holding portion 74 and oriented in the direction away from the mouthpiece 60. The holding portion 74 therefore comes into contact with a stepped portion of the groove portion 62 in the inner circumferential portion of the mouthpiece 60, as shown in Fig. 30. At this point, since the liquid is forcibly delivered into the channel in the endoscope 1, the entire channel is filled with the liquid.

On the other hand, when the endoscope 1, that is, the mouthpiece 60 is immersed in the liquid, and the supply of the liquid through the liquid delivery connector 50 is terminated, the pressure of the liquid in the groove portion 62 becomes equal to that in the environment, whereby the holding portion 74 can freely move in the groove portion 62. As a result, a gap is created between the inner circumferential portion of the mouthpiece 60 and the holding portion 74, and the entire inner circumferential portion of the mouthpiece 60 is immersed in the liquid, as shown in Fig. 29.

As described above, when the cleaning/disinfecting tube 70 is used to connect the liquid delivery connector 50 to the opening portion 61 of the mouthpiece 60 of the endoscope 1 in such a way that they communicate with each other, the liquid can be supplied into the channel, which communicates with the mouthpiece 60, and the entire mouthpiece 60, to which the tube connector section 71 is connected, can be immersed in the liquid in a reliable manner.

An eighth tube not part of the present invention will be described below. Fig. 31 is a view showing a hole portion in a distal end portion of an ultrasound probe. Fig. 32 is a view showing a cleaning/disinfecting tube of the eighth embodiment.

As shown in Fig. 31, an ultrasound probe 200 has a hole portion 201 in a distal end portion in some cases. The hole portion 201 is provided to allow, for example, a guide wire to pass therethrough. In processes of cleaning and disinfecting the ultrasound probe 200 in the endoscope cleaning/disinfecting apparatus, it is difficult to connect a cleaning/disinfecting tube, through which a liquid is supplied into the hole portion 201, to the hole portion 201 because the diameter thereof is small. Further, connecting the cleaning/disinfecting tube to the hole portion 201 disadvantageously results in an external force to be applied to the distal end portion of the ultrasound probe 200.

In the present tube, a liquid is supplied into the cleaning/disinfecting tube, into which the ultrasound probe 200 inserted, to clean and disinfect the ultrasound probe 200, as shown in Fig. 32.

A probe insertion guiding member 211 has one end connected to a first tube 210b into which a cleaning liquid is injected and a proximal end connected to a second tube 210a. A hollow path in the first tube 210b and a hollow path in the second tube 210a communicate with each other via a hollow path in the probe insertion guiding member 211. An insertion port 212 through which the ultrasound probe 200 is inserted is provided to the hollow path in the probe insertion guiding member 211.

The inner diameters of the hollow path in the probe insertion guiding member 211 and the hollow path in the second tube 210a are greater than the outer diameter of the ultrasound probe 200. The ultrasound probe 200 can therefore be inserted into the hollow paths in the probe insertion guiding member 211 and the second tube 210a through the insertion port 212.

When a liquid is supplied through the first tube 210b after the ultrasound probe 200 is inserted into the hollow paths in the probe insertion guiding member 211 and the second tube 210a, the liquid flows around the ultrasound probe 200 and through the hole portion 201.

According to the cleaning/disinfecting tube using the thus configured probe insertion guiding member, the liquid can be reliably supplied into the hole portion 201 having a small diameter without applying external force to the ultrasound probe 200.

The present invention is not limited to the embodiments described above but can be changed as appropriate to the extent that the changes do not depart from the substance of the present invention that one can read from the entire claims and specification, and cleaning/disinfecting tubes and endoscope cleaning/disinfecting apparatus in which such changes are made are also encompassed within the technical scope of the present invention.

## Claims

1. A cleaning/disinfecting tube (110, 110b) that allows an opening portion provided in an object (100) to be cleaned accommodated in a cleaning tank (2) of an endoscope cleaning/disinfecting apparatus to communicate with a liquid delivery connector (50) which is provided in the endoscope cleaning/disinfecting apparatus and through which a liquid is supplied, the cleaning/disinfecting tube (110, 110b) comprising:
a tube connector section (112, 113) to be connected to the opening portion; and
a holding portion (114) that engages with the object (100) to be cleaned with a gap therebetween and constrains a movement of the tube connector section (112, 113) in a direction away from the opening portion when the tube connector section (112, 113) is connected to the opening portion,
wherein the object (100) to be cleaned is a sliding tube into which an insertion section of an endoscope (1) is insertable, and
the holding portion (114) is configured to engage with an outer circumferential portion of the sliding tube,
wherein the holding portion (114) engages with a stepped portion (102) of the outer circumferential portion of the sliding tube, or
wherein the holding portion (114) frictionally engages with the outer circumferential portion of the sliding tube, **characterized by**:
a flexible connecting portion (115), one end of which is connected to the holding portion (114); and
a latching portion (116) disposed at a base portion of the tube connector section (112, 113) to which another end of the flexible connecting portion (115) is connected.

2. The cleaning/disinfecting tube (110, 110b) according to claim 1,
wherein a length of the connecting portion (115) along with the holding portion (114) defines a distance by which the tube connector section (112, 113) is spaced apart from the opening portion.

3. The cleaning/disinfecting tube (110, 110b) according to claim 2,
wherein the length of the connecting portion (115) does not change even when the connecting portion (115) is twisted.

4. An endoscope cleaning/disinfecting apparatus comprising the cleaning/disinfecting tube (110, 110b) according to any one of claims 1 to 3.

## Patentansprüche

1. Reinigungs-/Desinfektionsrohr (110, 110b), das es einem Öffnungsabschnitt, der in einem zu reinigendem Objekt (100) vorgesehen ist, welches in einem Reinigungsbehälter (2) eines Endoskop-Reinigungs-/Desinfektionsgeräts gelagert ist, ermöglicht, mit einem Flüssigkeitsversorgungsverbindungsstück (50) zu kommunizieren, das in dem Endoskop-Reinigungs-/Desinfektionsgerät vorgesehen ist, und durch das eine Flüssigkeit zugeführt wird, wobei das Reinigungs-/Desinfektionsrohr (110, 110b) aufweist:
einen Rohverbindungsabschnitt (112, 113), der zur Verbindung mit dem Öffnungsabschnitt vorgesehen ist; und
einen Halteabschnitt (114), der mit dem zu reinigenden Objekt (100) zusammenwirkt, wobei dazwischen ein Spalt vorhanden ist, und der eine Bewegung des Rohrverbindungsabschnitts (112, 113) in einer Richtung weg von dem Öffnungsabschnitt einschränkt, wenn der Rohrverbindungsabschnitt (112, 113) mit dem Öffnungsabschnitt verbunden ist,
wobei das zu reinigende Objekt (100) ein Gleitrohr ist, in das ein Einführabschnitt eines Endoskops (1) einführbar ist, und
der Halteabschnitt (114) dazu ausgelegt ist, mit einem äußeren Umfangsabschnitt des Gleitrohrs zusammenzuwirken,
wobei der Halteabschnitt (114) mit einem Stufenabschnitt (102) des äußeren Umfangsabschnitts des Gleitrohrs zusammenwirkt, oder
wobei der Halteabschnitt (114) eine Reibverbindung mit dem äußeren Umfangsabschnitt des Gleitrohrs eingeht,
**gekennzeichnet durch**:
einen flexiblen Verbindungsabschnitt (115), wobei ein Ende desselben mit dem Halteabschnitt (114) verbunden ist; und
einen Rastabschnitt (116), der an einem Basisabschnitt des Rohrverbindungsabschnitts (112, 113) vorgesehen ist und mit dem ein anderes Ende des flexiblen Verbindungsabschnitts (115) verbunden ist.

2. Reinigungs-/Desinfektionsrohr (110, 110b) gemäß Anspruch 1,
wobei eine Länge des Verbindungsabschnitts (115) zusammen mit dem Halteabschnitt (114) eine Strecke definiert, mit der der Rohrverbindungsabschnitt (112, 113) von dem Öffnungsabschnitt beabstandet ist.

3. Reinigungs-/Desinfektionsrohr (110, 110b) gemäß Anspruch 2,
wobei die Länge des Verbindungsabschnitts (115) sich nicht verändert, selbst wenn der Verbindungsabschnitt (115) verdreht wird.

4. Endoskop-Reinigungs-/Desinfektionsgerät, mit dem Reinigungs-/Desinfektionsrohr (110, 110b) gemäß einem der Ansprüche 1 bis 3.

## Revendications

1. Tube de nettoyage/désinfection (110, 110b) qui permet qu'une partie d'ouverture prévue dans un objet (100) devant être nettoyé reçu dans un bac de nettoyage (2) d'un appareil de nettoyage/désinfection d'endoscope communique avec un connecteur de délivrance de liquide (50) qui est prévu dans l'appareil de nettoyage/désinfection d'endoscope et à travers lequel un liquide est délivré, le tube de nettoyage/désinfection (110, 110b) comprenant :
une section de connecteur de tube (112, 113) destinée à être connectée à la partie d'ouverture ; et
une partie de maintien (114) qui s'engage avec l'objet (100) devant être nettoyé avec un espace entre ceux-ci et contraint un mouvement de la section de connecteur de tube (112, 113) dans un sens à l'opposé de la partie d'ouverture lorsque la section de connecteur de tube (112, 113) est connectée à la partie d'ouverture,
dans lequel l'objet (100) devant être nettoyé est un tube coulissant dans lequel une section d'insertion d'un endoscope (1) est insérable, et
la partie de maintien (114) est configurée pour s'engager avec une partie circonférentielle extérieure du tube coulissant,
dans lequel la partie de maintien (114) s'engage avec une partie épaulée (102) de la partie circonférentielle extérieure du tube coulissant, ou
dans lequel la partie de maintien (114) s'engage par frottement avec la partie circonférentielle extérieure du tube coulissant, **caractérisé par** :
une partie de connexion (115) flexible, une extrémité de laquelle est connectée à la partie de maintien (114) ; et
une partie de verrouillage (116) disposée au niveau d'une partie de base de la section de connecteur de tube (112, 113) à laquelle une autre extrémité de la partie de connexion (115) flexible est connectée.

2. Tube de nettoyage/désinfection (110, 110b) selon la revendication 1,
dans lequel une longueur de la partie de connexion (115) avec la partie de maintien (114) définit une distance de laquelle la section de connecteur de tube (112, 113) est espacée de la partie d'ouverture.

3. Tube de nettoyage/désinfection (110, 110b) selon la revendication 2,
dans lequel la longueur de la partie de connexion (115) ne change pas, même lorsque la partie de connexion (115) est vrillée.

4. Appareil de nettoyage/désinfection d'endoscope comprenant le tube de nettoyage/désinfection (110, 110b) selon l'une quelconque des revendications 1 à 3.
